Europäisches Patentamt

European Patent Office

Office européen des brevets

(19)

(11) Veröffentlichungsnummer: **0 379 644**
**A1**

## (12) EUROPÄISCHE PATENTANMELDUNG

(21) Anmeldenummer: **89117928.5**

(22) Anmeldetag: **28.09.89**

(51) Int. Cl.5: **G01N 33/28**

(30) Priorität: **22.12.88 DE 3843177**

(43) Veröffentlichungstag der Anmeldung:
**01.08.90 Patentblatt 90/31**

(84) Benannte Vertragsstaaten:
**DE FR GB IT SE**

(71) Anmelder: **FEV Motorentechnik GmbH & Co. KG**
**Jülicher Strasse 342-352**
**D-5100 Aachen(DE)**

(72) Erfinder: **Scheid, Ernst, Dr.-Ing.**
**Soerser Winkel 31**
**D-5100 Aachen(DE)**

(74) Vertreter: **Fischer, Friedrich B., Dr.-Ing.**
**Saarstrasse 71**
**D-5000 Köln 50 (Rodenkirchen)(DE)**

(54) Verfahren zur Feststellung des Alkoholgehaltes und/oder des Heizwertes von Kraftstoffen.

(57) Bei einem Verfahren zur Feststellung des Alkoholgehaltes und/oder des Heizwertes von Kraftstoffen durch Messung elektrisch meßbarer Größen des Kraftstoffs wird eine Verbesserung der Meßgenauigkeit und eine apparative Vereinfachung dadurch erreicht, daß bei Brennkraftmaschinen wenigstens ein Bauteil des Kraftstoffsystems gegenüber einer Gegenelektrode zur kondensatorischen Dielektrizitätsmessung des Kraftstoffs eingesetzt wird. Dabei kann es vorteilhaft sein, daß zusätzlich ein Bauteil des Kraftstoffsystems gegenüber einer Gegenelektrode zur Messung des Leitwertes des Kraftstoffs einge- setzt wird. Auch kann die gleiche Gegenelektrode zur Dielektrizitätsmessung und zur Messung des Leitwertes des Kraftstoffs eingesetzt werden.

Fig. 1

EP 0 379 644 A1

## Verfahren zur Feststellung des Alkoholgehaltes und/oder des Heizwertes von Kraftstoffen

Die Erfindung bezieht sich auf ein Verfahren zur Feststellung des Alkoholgehaltes und/oder des Heizwertes von Kraftstoffen durch Messung elektrisch meßbarer Größen des Kraftstoffs.

In Anbetracht der langfristig nur in begrenztem Umfang verfügbaren Reserven an fossiler Energie, insbesondere der aus Rohöl gewonnenen Kraftstoffe, und in Anbetracht der steigenden Anforderungen an den Umweltschutz, wird diesen Kraftstoffen in zunehmendem Maße Methyl- oder Äthylalkohol zugemischt. Dabei soll ein beliebiges Tanken sowohl von Reinkraftstoffen als auch von Mischkraftstoffen möglich sein. Bei höheren Alkoholanteilen ist dabei die Kenntnis des Mischungsverhältnisses erforderlich, um eine optimale Arbeitsweise der Brennkraftmaschine zu erreichen und dabei insbesondere eine genaue, den Betriebsverhältnissen angepaßte Kraftstoffzumessung zu ermöglichen. Besondere Probleme bereitet dabei die laufende Feststellung des Alkoholgehaltes des der Brennkraftmaschine im Betrieb zugeführten Kraftstoffs bei Fahrzeugmotoren, bei denen durch das beliebige Tanken der Kraftstoffarten jede mögliche Mischung erreicht werden kann.

Die bekannten optischen Verfahren sind zu diesem Zweck kaum geeignet, da sie meist Grenzflächeneffekte zur Bestimmung des Brechungsindexes ausnutzen, aus dem dann auf den Alkoholanteil geschlossen werden kann. Abgesehen von der Schwierigkeit der Auswertung bei Fahrzeugmotoren ist ein Nachteil dieses Verfahrens auch, daß die messend zu beobachtende Mischung eine hohe Homogenität aufweisen muß, die insbesondere auch an der Grenz fläche vorhanden sein muß. Mit diesem Verfahren wurden nicht die erforderlichen Genauigkeiten erreicht.

In der Schrift "Proceedings of the Fourth International Symposium on Alcohol Fuels Technology", Sao Paulo, Brasilien, vom 05.10.1980, wird die Möglichkeit der Feststellung des Alkoholgehaltes von Kraftstoffen durch Dielektrizitätsmessungen beschrieben. Das Verfahren wurde jedoch verworfen, da eine für Verbrennungsmotoren geeignete zuverlässige Messung nicht durchgeführt werden konnte. Einer der Gründe hierfür dürfte sein, daß die meßtechnische Realisierung der Dielektrizitätsmessung erhebliche Schwierigkeiten bereitete.

Der Erfindung liegt daher die Aufgabe zugrunde, ein Verfahren der eingangs bezeichneten Art zu schaffen, das insbesondere in der Anwendung auf Fahrzeugmotoren eine genaue und zuverlässige Feststellung des Alkoholgehaltes und/oder des Heizwertes von Kraftstoffen erlaubt, und das zugleich in einfacher und kostengünstiger Weise in Brennkraftmaschinen meßtechnisch ausgeübt werden kann.

Gemäß der Erfindung ist bei einem Verfahren der eingangs bezeichneten Art vorgesehen, daß bei Brennkraftmaschinen wenigstens ein Bauteil des Kraftstoffsystems gegenüber einer Gegenelektrode zur kondensatorischen Dielektrizitätsmessung des Kraftstoffs eingesetzt wird. Dabei ist es zweckmäßig, daß zusätzlich ein Bauteil des Kraftstoffsystems gegenüber einer Gegenelektrode zur Messung des Leitwert des Kraftstoffs eingesetzt wird, um Quereinflüsse auszuschalten, die insbesondere durch Wasseranteile und andere Verschmutzungen oder Beimischungen des Kraftstoffs verursacht sind.

Dadurch, daß zur kondensatorischen Dielektrizitätsmessung des Kraftstoffs ein Bauteil des Kraftstoffsystems eingesetzt wird, ist in vorteilhafter Weise erreicht, daß der aus Bauteil und Gegenelektrode mit Kraftstoff als Dielektrikum bestehenden Meßkondensator-Anordnung verhältnismäßig große Kondensatorflächen zur Verfügung stehen, die eine wesentlich genauere Dielektrizitätsmessung erlauben als separate Meßanordnungen herkömmlicher Art. Zugleich werden hierdurch Kosten für separate Meßanordnungen eingespart. Ein weiterer Vorteil ist, daß nicht nur eine Platzersparnis erreicht wird, sondern auch, daß die Meßanordnung mechanisch und gegenüber elektrischen Störfeldern besser geschützt ist.

Gemäß einer bevorzugten Ausführungsform ist vorgesehen, daß die gleiche Gegenelektrode zur Dielektrizitätsmessung und zur Messung des Leitwerts des Kraftstoffs eingesetzt wird.

Als Bauteil des Kraftstoffsystems zur kondensatorischen Dielektrizitätsmessung wird vorzugsweise das Leitungssystem des Kraftstoffs benutzt, und zwar insbesondere eine metallische Leitung, jedoch können auch Vorteile erreicht werden, wenn bei einer nichtmetallischen Kraftstoffleitung eine außenliegende Elektrode angeordnet wird, da auch in einem solchen Fall eine relativ große Kondensatorfläche zur Verfügung steht. Im übrigen werden vor allem auch kraftstoffspeichernde Teile des Kraftstoffsystems zur kondensatorischen Dielektrizitätsmessung verwendet werden, z.B. der Kraftstofftank, der Druckregler, das Druckhalteventil oder der Federspeicher.

Ausführungsbeispiele der Erfindung werden nachfolgend anhand der Zeichnung näher beschrieben.

Fig. 1 zeigt im Längsschnitt ein Beispiel für die Ausübung des Verfahrens in einem unmittelbar vor den Kraftstoffeinspritzventilen einer Brennkraftmaschine angeordneten Kraftstoffverteilerrohr.

Fig. 2 zeigt vereinfacht und geschnitten die

Anwendung des Verfahrens bei einem Federspeicher eines Kraftstoffsystemß.

Fig. 3 zeigt im Längsschnitt die Anwendung des Verfahrens im Kraftstoffleitungssystem einer Brennkraftmaschine.

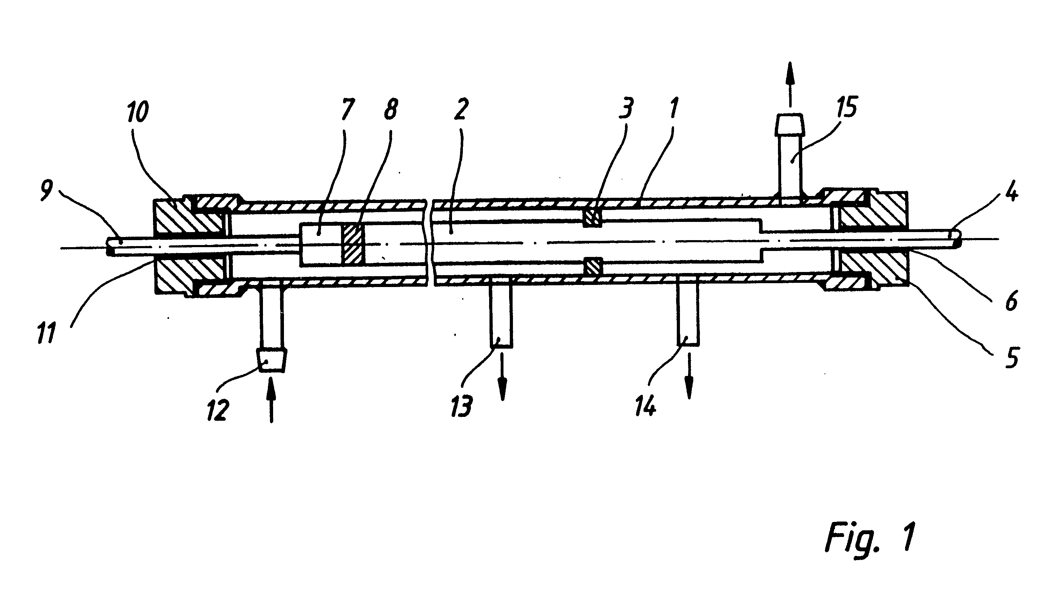

In Fig. 1 ist eine bevorzugte Ausführungsform einer Meßeinrichtung zur Ausübung des Verfahrens der kondensatorischen Messung der Dielektrizitätskonstanten dargestellt. Die Meßeinrichtung besteht im wesentlichen aus dem unmittelbar vor den Kraftstoffeinspritzventilen einer Einspritzbrennkraftmaschine angeordneten Kraftstoffverteilerrohr 1, das als Teil des Kraftstoffsystems die äußere Elektrode eines Kondensators zur Messung der Dielektrizitätskonstanten des Kraftstoffs bildet. Innerhalb des aus einem elektrisch leitfähigen Material hergestellten Verteilerrohrs 1 befindet sich eine zylindrische Gegenelektrode 2, die durch Abstandshalter 3 in einem konstanten Abstand zu dem als Außenelektrode wirkenden Verteilerrohr 1 gehalten wird.

Der elektrische Anschluß 4 der Gegenelektrode 2 zur Messung der Dielektrizitätskonstanten ist durch eine Verschraubung 5 mit Isolation 6 nach außen geführt und kann unmittelbar mit einer (nicht dargestellten) elektronischen Meßschaltung verbunden werden.

Gemäß einer bevorzugten Ausführungsform ist zusätzlich eine Elektrode 7 zur Messung der Leitfähigkeit des Kraftstoffs vorhanden, die ebenfalls die Funktion einer Gegenelektrode zum Kraftstoffverteilerrohr 1 als erster Elektrode übernimmt. Bei dem dargestellten Ausführungsbeispiel ist die Elektrode 7 ebenso wie die Gegenelektrode 2 zylindrisch ausgebildet, und sie ist von dieser durch eine Isolation 8 getrennt. Ihre Abmessung kann im allgemeinen erheblich geringer sein als die der Gegenelektrode 2.

Der elektrische Anschluß 9 der Elektrode 7 zur Messung der Leitfähigkeit kann in ähnlicher Weise wie bei der Gegenelektrode 2 durch eine Verschraubung 10 mit Isolation 11 nach außen geführt und mit einer Meßschaltung verbunden werden. Das Kraftstoffverteilerrohr 1 dient dabei als gemeinsame Elektrode sowohl für die Dielektrizitätsmessung als auch für die Leitfähigkeitsmessung.

Der Kraftstoff strömt bei Kraftstoffanschluß 12 in das Kraftstoffverteilerrohr 1 ein, und er kann bei Leitungsanschlüssen 13 und 14 zu den (nicht dargestellten) Kraftstoffeinspritzventilen gelangen. Der übrige Kraftstoff gelangt zum Kraftstoffrücklauf 15.

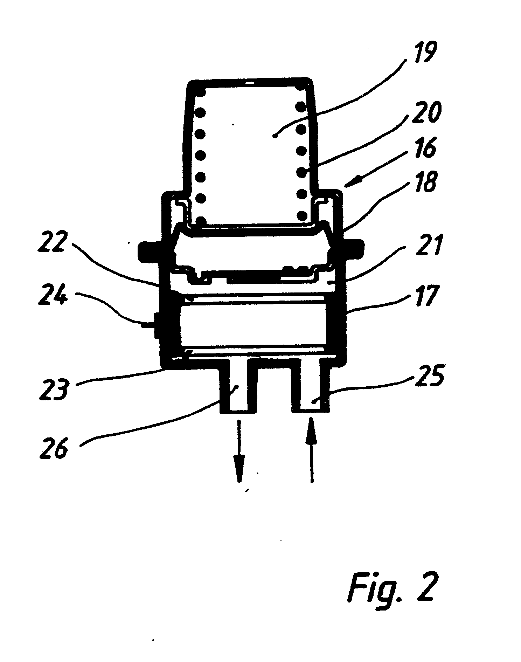

Ein weiteres Ausführungsbeispiel des Verfahrens zur kondensatorischen Messung der Dielektrizitätskonstanten des Kraftstoffs ist in Fig. 2 dargestellt. Hier ist als Bauteil des Kraftstoffsystems ein Federspeicher 16 zur Aufrechterhaltung eines bestimmten Kraftstoffvordrucks im Kraftstoffsystem mit einer Innenelektrode 17 ausgerüstet. Der Federspeicher 16 ist durch eine Membran 18 aufgeteilt in eine Federkammer 19 mit Feder 20 und einen von Kraftstoff durchströmten Raum 21, in dem sich die Innenelektrode 17 befindet. Die Innenelektrode 17 ist durch Halterungen 22 und 23 gegenüber dem leitfähigen Gehäuse isoliert gelagert. Der elektrische Anschluß 24 der Innenelektrode 17 ist isoliert aus dem Gehäuse herausgeführt und kann unmittelbar mit einer Meßschaltung verbunden werden. Der Kraftstoff tritt über Kraftstoffeintrittsstutzen 25 in das Gehäuse des Federspeichers 16 ein, und er wird über Kraftstoffaustrittsstutzen 26 weitergeleitet.

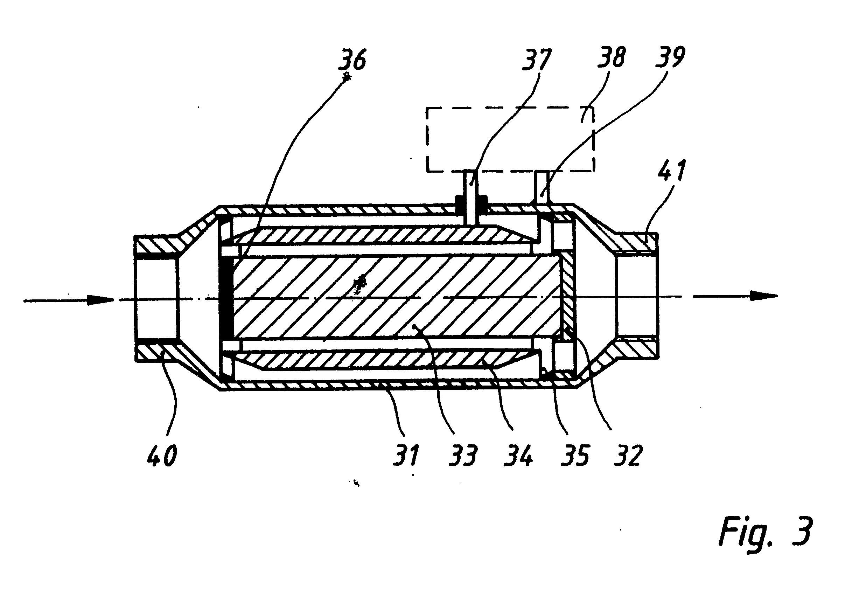

Die in Fig. 3 dargestellte Ausführungsform ermöglicht im Vergleich zu den in den Figuren 1 und 2 dargestellten Anordnungen die Ausbildung einer noch größeren Kondensatorfläche, so daß eine höhere Kapazität und damit auch eine verbesserte Meßgenauigkeit erreichbar ist. Durch die erhöhte Kondensatorkapazität wird in vorteilhafter Weise auch die Auslegung der elektronischen Meßschaltung erleichtert.

Die äußere Elektrode 31 ist ein Teil des Kraftstoffsystems, beispielsweise eine Rohrleitung, die erforderlichenfalls auch geringfügig erweitert sein kann. Die äußere Elektrode 31 ist über einen Abstandshalter 32 mit einem zylindrischen Innenteil 33 elektrisch leitend verbunden. Die Gegenelektrode zu den Elektroden 31 und 33 bildet ein Hohlzylinder 34, der in Abstandshaltern 35 und 36 isoliert gelagert ist. Die hohlzylindrische Elektrode 34 ist über einen isolierten Anschluß 37 mit einer elektronischen Meß- und/oder Auswerteschaltung 38 verbunden. Die Elektroden 31 und 33 sind über Anschluß 39 mit der Meß- und/oder Auswerteschaltung verbunden.

Der Kraftstoff tritt über Eintrittsstutzen 40 in die Meßanordnung ein, und der Kraftstoffstrom verzweigt sich in der dargestellten Weise auf beide Seiten des Hohlzylinders 34. Der Kraftstoff wird über Austrittsstutzen 41 weitergeleitet.

**Ansprüche**

1. Verfahren zur Feststellung des Alkoholgehaltes und/oder des Heizwertes von Kraftstoffen durch Messung elektrisch meßbarer Größen des Kraftstoffs, dadurch gekennzeichnet, daß bei Brennkraftmaschinen wenigstens ein Bauteil des Kraftstoffsystems gegenüber einer Gegenelektrode zur kondensatorischen Dielektrizitätsmessung des Kraftstoffs eingesetzt wird.

2. Verfahren nach Anspruch 1, dadurch gekennzeichnet, daß zusätzlich ein Bauteil des Kraftstoffsystems gegenüber einer Gegenelektrode zur Messung des Leitwertes des Kraftstoffs eingesetzt wird.

3. Verfahren nach Anspruch 1 oder 2, dadurch gekennzeichnet, daß die gleiche Gegenelektrode zur Dielektrizitätsmessung und zur Messung des Leitwertes des Kraftstoffs eingesetzt wird.

4. Verfahren nach einem der Ansprüche 1-3, dadurch gekennzeichnet, daß als Bauteil des Kraftstoffsystems das Kraftstoffleitungssystem eingesetzt wird.

5. Verfahren nach einem der Ansprüche 1-4, dadurch gekennzeichnet, daß als Bauteil des Kraftstoffsystems eine nichtmetallische Kraftstoffleitung mit wenigstens einer außenliegenden Elektrode eingesetzt wird.

6. Verfahren nach einem der Ansprüche 1-3, dadurch gekennzeichnet, daß als Bauteil des Kraftstoffsystems kraftstoffspeichern de Teile (Kraftstofftank, Druckregler, Druckhalteventil, Federspeicher) eingesetzt werden.

Fig. 1

Fig. 2

Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | PATENT ABSTRACTS OF JAPAN, Band 5, Nr. 175 (P-88)[847], 11. November 1981; & JP-A-56 104 243 (HITACHI SEISAKUSHO K.K.) 19-08-1981 --- | 1,4 | G 01 N 33/28 |
| Y | IDEM --- | 3,2 | |
| Y | US-A-2 892 976 (THAYER) * Spalte 5, Zeilen 34-45; Figur 4 * --- | 3,2 | |
| A | FR-A-2 409 389 (TNO) * Seite 5, Zeile 28 - Seite 6, Zeile 13; Figur 2 * ----- | 2 | |

| | | | RECHERCHIERTE SACHGEBIETE (Int. Cl.5) |
|---|---|---|---|
| | | | G 01 N |

Der vorliegende Recherchenbericht wurde für alle Patentansprüche erstellt

| Recherchenort | Abschlußdatum der Recherche | Prüfer |
|---|---|---|
| DEN HAAG | 26-04-1990 | BOSMA R.A.P. |